# EUROPÄISCHE PATENTANMELDUNG

(11) **EP 0 527 364 A2**
(43) Veröffentlichungstag der Anmeldung: **17.02.1993**
(21) Anmeldenummer: 92112533.2
(22) Anmeldetag: 01.08.1992
(51) Int. Cl.: C07C 213/06, C07C 219/06

(54) **Verfahren zur Herstellung von Succinylcholinhalogeniden**

(30) Priorität: 13.08.1991 AT 1591/91
(71) Anmelder: Hafslund Nycomed Pharma Aktiengesellschaft, A-4021 Linz (AT)
(72) Erfinder: Raml, Walter, Dr., A-4202 Hellmonsödt (AT); Eichberger, Günther, Dr., A-4512 Weisskirchen (AT)

(57) **Zusammenfassung**

Herstellung von Succinylcholinhalogeniden der Formel I 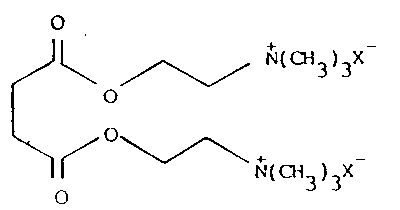
durch Umsetzung von Bernsteinsäuredialkylestern mit einem Überschuß an Dimethylaminoethanol in Gegenwart eines Alkalialkoholat- oder Alkaliamidkatalysators und anschließender Reaktion der so hergestellten Bernsteinsäure-bis-(2-dimethylaminoethylester) mit Methylhalogeniden.

## Beschreibung

Die Erfindung betrifft ein Verfahren zur Herstellung von Succinylcholinhalogeniden.

Succinylcholinhalogenide stellen pharmazeutisch wirksame Verbindungen dar, die als Muskelrelaxans Verwendung finden.
Aus der Literatur sind bereits verschiedene Verfahren zur Herstellung von Succinylcholinhalogeniden bekannt.
Im Bulletin of the Institute of Chemistry, Academia Sinica, 26 (1979), Seiten 47-54 werden 5 Methoden zur Herstellung von Succinylcholinchlorid beschrieben:
Nach Methode I läßt man Bernsteinsäure mit Dimethylaminoethanol zu Bernsteinsäure-bis-(2-dimethylaminoethanolester) reagieren und setzt diesen dann mit Methylchlorid zum Succinylcholinchlorid um.
Methode II geht von Bernsteinsäure-bis-(2-chlorethylester) aus, der mit Dimethylamin umgesetzt wird. Anschließend erhält man wiederum durch Reaktion mit Methylchlorid Succinylcholinchlorid.
Bei beiden Methoden ist die Ausbeute jedoch relativ gering Methode III beschreibt die Reaktion von Bernsteinsäure mit Ethylenchlorhydrin zu Bernsteinsäue-bis-(2-chlorethylester) und anschließender Alkylierung mit Trimethylamin, wobei Succinylcholinchlorid ebenfalls nur in geringer Ausbeute erhalten wird.
Nach Methode IV erhält man Succinylcholinchlorid durch Reaktion von Bernsteinsäuredichlorid mit Cholinchlorid. Diese Methode hat jedoch den Nachteil, daß beide Ausgangsprodukte sehr hygroskopisch und somit schwer handhabbar sind und außerdem ein zusätzlicher Schritt zur Herstellung des Säurechlorides aus Bernsteinsäureanhydrid mit Thionylchlorid benötigt wird.

Methode V geht vom Bernsteinsäureanhydrid aus, das in Benzol mit Cholinchlorid in Gegenwart von trockener HCl als Katalysator umgesetzt wird. Auch hier muß das hygroskopische Cholinchlorid verwendet werden. Ein weiterer Nachteil ist das Arbeiten mit Benzol.

Aus J.Am.Chem.Soc. (1949) 149, Seite 3264 ist ein Verfahren zur Herstellung von Bis-(β-dimethylaminoethyl)estern von aliphatischen Dicarbonsäuren durch Umsetzung der Methyl- oder Ethylester mit einem geringen Überschuß an Dimethylaminoethanol in Gegenwart von geringen Mengen an gelöstem Natrium bekannt. Die gewünschten Aminoester werden dabei aber nur in geringen Ausbeuten erhalten.

Es wurde nun unerwarteter Weise ein Verfahren zur Herstellung von Succinylcholinhalogeniden gefunden, bei dem ausgehend von Bernsteinsäuredialkylestern und Dimethylaminoethanol, das gleichzeitig als Verdünnungsmittel dient, in Gegenwart eines Alkalialkoholat- oder Alkaliamidkatalysators und anschließender Reaktion mit Methylhalogeniden Succinylcholinhalogenide mit hoher Ausbeute und hoher Reinheit erhalten werden.
Gegenstand der Erfindung ist demnach ein Verfahren zur Herstellung von Succinylcholinhalogenid der Formel I 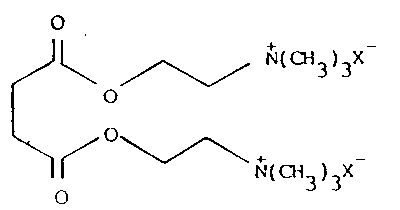
in der X Chlor, Brom, Jod bedeutet,
dadurch gekennzeichnet, daß Bernsteinsäuredialkylester der Formel II 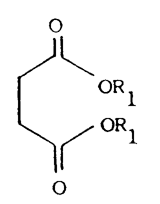
in der R₁ (C₁ - C₄)-Alkyl bedeutet, in Gegenwart von Alkalialkoholaten oder -amiden als Katalysator mit einem Überschuß an Dimethylaminoethanol versetzt wird, wobei der bei der Reaktion entstehende Alkohol laufend abdestilliert, überschüssiges Dimethylaminoethanol rückgewonnen, anschließend der Katalysator inaktiviert und abfiltriert wird, worauf der entstandene Bernsteinsäure-bis-(2-dimethylaminoethylester) der Formel III 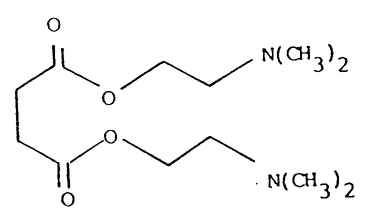
mit Methylhalogeniden zu einer Verbindung der Formel I umgesetzt wird.

Zur Durchführung des erfindungsgemäßen Verfahrens wird zuerst Dimethylaminoethanol, das gleichzeitig als Verdünnungsmittelmittel dient, in einem mit N₂ gespülten Reaktionsgefäß vorgelegt und der Katalysator auf einmal dazugegeben.
Pro Mol Bernsteinsäuredialkylester werden etwa 2,5 bis 20 Äquivalente, das sind 5 bis 40 Mol Dimethylaminoethanol, vorzugsweise 7 bis 10 Mol, besonders bevorzugt 8 bis 9 Mol, eingesetzt. Die Verwendung größerer Mengen Dimethylaminoethanol hat keine negativen Auswirkungen auf die Reaktion, man muß nach Beendigung der Reaktion jedoch mehr Dimethylaminoethanol abdestillieren. Als Katalysator werden Alkalialkoholate oder -amide, beispielsweise Lithiummethanolat, Lithiumethanolat, Lithiumamid, Natriummethanolat, Natriumamid, Kaliumamid oder Kaliummethanolat eingesetzt. Bevorzugt werden Lithiummethanolat, Lithiumamid oder Natriummethanolat verwendet. Die Katalysatormenge beträgt etwa 0,5 bis 6 Gewichtsprozent, bezogen auf Bernsteinsäuredialkylester, vorzugsweise etwa 1 bis 3 Gewichtsprozent, besonders bevorzugt etwa 2 Gewichtsprozent.
Anschließend wird auf etwa 30 bis 120 °C, bevorzugt auf etwa 75 bis 90 °C, erwärmt und Bernsteinsäuredialkylester innerhalb von etwa 15 bis 30 Minuten dazugegeben. Als Bernsteinsäuredialkylester werden Ester mit 1 bis 4 C-Atomen, vorzugsweise mit 1 bis 2 C-Atomen in der Alkylkette eingesetzt. Der Reaktionsdruck kann zwischen 50 und 760 mbar, bevorzugt zwischen 100 und 300 mbar, liegen. Es ist von Vorteil, den Druck zu senken, etwa auf 200 mbar, da so der bei der Reaktion entstehende Alkohol leichter und schneller abdestilliert werden kann.
Der entstehende Alkohol wird laufend abdestilliert, wobei die Hauptmenge des Alkohols innerhalb der ersten 15 bis 30 Minuten abgetrennt wird.
Nach beendeter Umsetzung wird der Druck weiter abgesenkt und überschüssiges Dimethylaminoethanol abdestilliert.
Das so zurückgewonnene Dimethylaminoethanol kann wieder als Ausgangsverbindung bzw. als Verdünnungsmittel eingesetzt werden. Nach dem Abdestillieren von Dimethylaminoethanol wird mit N₂ belüftet und der Katalysator inaktiviert, etwa indem eine der Katalysatormenge äquivalente Menge einer organischen Säure, wie z.B. Essigsäure, Oxalsäure, Ameisensäure, Bernsteinsäure der Reaktionslösung zugegeben wird.
Um den dabei anfallenden gelartigen Niederschlag besser abfiltrieren zu können, versetzt man vorzugsweise das Reaktionsgemisch mit einem inerten Verdünnungsmittel, das nach dem Abfiltrieren des entstandenen Alkalisalzes leicht wieder entfernt und wiedergewonnen werden kann. Es können unter anderem Benzine, Toluol oder Ether, wie z.B. Diisopropylether verwendet werden. Bevorzugt wird Diisopropylether eingesetzt. Der verbleibende Rückstand an Bernsteinsäure-bis-(2-dimethylaminoethylester) wird dann in üblicher Weise gereinigt. Besonders geeignete Reinigungsmethoden stellen Dünnschichtverdampfen und Kurzwegdestillation dar.
Zur weiteren Umsetzung wird der Bernsteinsäure-bis-(2-dimethylaminoethylester) in einem inerten Verdünnungsmittel wie z.B. Aceton, Tetrahydrofuran oder Diisopropylether, suspendiert und gegebenenfalls über Aktivkohle geschönt. Vorzugsweise wird Aceton als Verdünnungsmittel verwendet. In die Suspension wird ein Methylhalogenid unter Druck eingeleitet. Pro Mol Bernsteinsäure-bis-(2-dimethylaminoethylester) werden 2 Mol Methylhalogenid benötigt. Bevorzugt werden 2,5 bis 9 Mol Methylhalogenid pro Mol Ester, besonders bevorzugt 2,8 bis 3,2 Mol Methylhalogenid pro Mol Ester, eingesetzt. Die Temperatur wird anschließend auf etwa 30 bis 100 °C, bevorzugt auf 40 bis 60 °C, erhöht. Nach Beendigung der Reaktion, üblicherweise nach etwa 1 bis 20 Stunden wird das Reaktionsgemisch abgekühlt und das Verdünnungsmittel und überschüssiges Methylhalogenid abdestilliert. Das zurückgewonnene Verdünnungsmittel sowie Methylhalogenid können wieder als Verdünnungsmittel bzw. als Quarternisierungsmittel eingesetzt werden.
Das erhaltene Succinylcholinhalogenid kann auf übliche Weise, beispielsweise aus einem Ethanol-Wasser-Gemisch, umkristallisiert werden.

Nach dem erfindungsgemäßen Verfahren werden Succinylcholinhalogenide in hohen Ausbeuten und in hoher Reinheit erhalten.

### Beispiel 1

### Herstellung von Bernsteinsäure-bis-(2-dimethylaminoethylester)

1418,56 g (15,9 Mol) Dimethylaminoethanol wurden in einem mit N₂ gespülten Reaktionsgefäß vorgelegt und 10 g (0,43 Mol) LiNH₂ zugegeben, worauf NH₃ entwich. Die Temperatur wurde auf ca. 70 °C erhöht, der Druck auf 200 mbar gesenkt und 500 g (3,42 Mol) Bernsteinsäuredimethylester innerhalb 20 Minuten zugegeben. Das durch die Reaktion entstandene Methanol wurde laufend abdestilliert, wobei der Großteil an Methanol (ca. 200 ml) in den ersten 15 Minuten abgetrennt wurde (theoretische Gesamtmenge an Methanol = 277 ml).
Nach etwa 7 Stunden wurde der Druck auf 15 mbar gesenkt und überschüssiges Dimethylaminoethanol und restliches Methanol abdestilliert. Anschließend wurde mit N₂ belüftet und das Reaktionsgemisch mit einer auf LiNH₂ bezogenen äquimolaren Menge an Essigsäure versetzt. Um den dadurch ausgefallenen gelartigen Lithiumacetatniederschlag leichter abfiltrieren zu können, wurden 2000 ml Diisopropylether bei ca. 50 - 60 °C zur Reaktioslösung zugegeben.
Der Ether wurde dann vom Filtrat abdestilliert und der verbleibende Bernsteinsäure-bis-(2-diemthylaminoethylester) Dünnschichtdestilliert.
Ausbeute: 806 g (90,64 %), Reinheit: 99,5 %

Es wurden eine Reihe weitere Versuche in analoger Weise durchgeführt. Die Daten sind aus Tabelle 1 ersichtlich.

**Tabelle 1**

| | **LiOCH₃** | **NaOCH₃** | **KOCH₃** |
|---|---|---|---|
| **Gew.%** | 2 | 3 | 6 |
| **T (°C)** | 65-87 | 65-80 | 65-88 |
| **Rohausbeute** | 96,6 | 87 | 99,9 |
| **Reinheit nach** | | | |
| **Destillation** | 99,5 | 99,2 | 99,5 |

### Beispiel 2

### Herstellung von Succinylcholinhalogenid

54 g (0,21 Mol) Bernsteinsäure-bis-(2-dimethylester) wurden in 700 ml Aceton gelöst und 27 g (0,54 Mol) CH₃Cl unter Druck eingeleitet. Nach ca. 8 Stunden bei 60 °C wurde das Reaktionsgemisch abgekühlt und Aceton und überschüssiges CH₃Cl abdestilliert. Das erhaltene Succinylcholinchlorid wurde in einem Ethanol/H₂O-Gemisch (80/20) umkristallisiert.
Ausbeute: 68 g (89,6 %), weißes, kristallines Pulver
Schmelzpunkt: 160 °C
Reinheit: Gehalt an Cholinchlorid < 0,5 %
Gehalt an Succinylmonocholinchlorid < 0,5 %

## Patentansprüche

1. Verfahren zur Herstellung von Succinylcholinhalogenid der Formel I 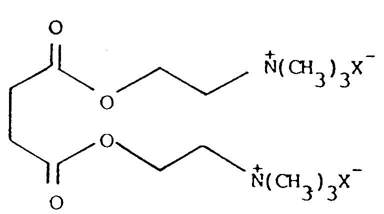 in der X Chlor, Brom, Jod bedeutet,
dadurch gekennzeichnet, daß Bernsteinsäuredialkylester der Formel II 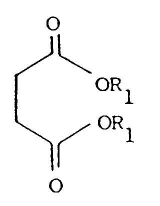 in der R₁ (C₁ - C₄)-Alkyl bedeutet, in Gegenwart von Alkalialkoholaten oder -amiden als Katalysator mit einem Überschuß an Dimethylaminoethanol versetzt wird, wobei der bei der Reaktion entstehende Alkohol laufend abdestilliert, überschüssiges Dimethylaminoethanol rückgewonnen, anschließend der Katalysator inaktiviert und abfiltriert wird, worauf der entstandene Bernsteinsäure-bis-(2-dimethylaminoethylester) der Formel III 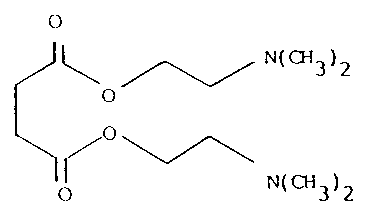 mit Methylhalogeniden zu einer Verbindung der Formel I umgesetzt wird.

2. Verfahren gemäß Anspruch 1, dadurch gekennzeichnet, daß als Katalysator Lithium- oder Natriummethanolat eingesetzt wird.

3. Verfahren gemäß Anspruch 1, dadurch gekennzeichnet, daß als Katalysator Lithiumamid eingesetzt wird.

4. Verfahren gemäß Anspruch 1, dadurch gekennzeichnet, daß die Katalysatormenge 0,5 bis 6 Gew.% bezogen auf die Ausgangsverbindung der Formel II, vorzugsweise 1 bis 3 Gew.% beträgt.

5. Verfahren gemäß Anspruch 1, dadurch gekennzeichnet, daß das Molverhältnis von der Verbindung der Formel II zu Dimethylaminoethanol zwischen 1 : 2,5 und 1 : 40, vorzugsweise zwischen 1 : 7 und 1 : 10, liegt.

6. Verfahren gemäß Anspruch 1, dadurch gekennzeichnet, daß die Reaktion von Dimethylaminoethanol mit der Verbindung der Formel II bei Temperaturen von 30 bis 120°C, vorzugsweise von 75 bis 90 °C, durchgeführt wird.

7. Verfahren gemäß Anspruch 1, dadurch gekennzeichnet, daß der Katalysator mit einer organischen Säure inaktiviert wird.
